# NEUE EUROPÄISCHE PATENTSCHRIFT

(11) **EP 2 430 062 B2**
(45) Veröffentlichungstag und Bekanntmachung des Hinweises auf die Entscheidung über den Einspruch: **10.07.2019**
(45) Hinweis auf die Patenterteilung: 07.09.2016
(21) Anmeldenummer: 10715279.5
(22) Anmeldetag: 03.05.2010
(51) Int. Cl.: C08G 18/02, C08G 18/76

(54) **VERFAHREN ZUR HERSTELLUNG VON CARBODIIMIDEN**
METHOD FOR PRODUCING CARBODIIMIDES
PROCÉDÉ DE PRODUCTION DE CARBODIIMIDES

(30) Priorität: 15.05.2009 DE 102009021602
(43) Veröffentlichungstag der Anmeldung: 21.03.2012
(73) Patentinhaber: LANXESS Deutschland GmbH, 50569 Köln (DE)
(72) Erfinder: BLAUL, Anke, 64297 Darmstadt (DE); LAUFER, Wilhelm, 67158 Ellerstadt (DE); KRAY, Bernd, 67346 Speyer (DE); WUEHR, Michael, 69493 Hirschberg (DE)
(86) Internationale Anmeldenummer: PCT/EP2010/055977
(87) Internationale Veröffentlichungsnummer: WO 2010/130594

(56) Entgegenhaltungen:
- EP-A1- 0 609 698
- EP-A2- 0 785 222
- EP-A2- 1 650 238
- US-A- 5 750 636

## Beschreibung

Die vorliegende Erfindung betrifft ein Verfahren zur Herstellung von Carbodiimiden. Weiterer Gegenstand der vorliegenden Erfindung sind die durch das erfindungsgemäße Verfahren erhältlichen Carbodiimide.

Die vorliegende Erfindung betrifft darüber hinaus Carbodiimide mit einem hohen Molekulargewicht und/oder geringer Polydispersität sowie die Verwendung von erfindungsgemäßen Carbodiimiden unter anderem als Stabilisatoren gegen den hydrolytischen Abbau von Estergruppen enthaltenden Verbindungen und als Vernetzer und Kettenverlängerer in Kunststoffen. Carbodiimide weisen bei höherem Molekulargewicht und bei vergleichbarem Molekulargewicht bei geringerer Polydispersität in diesen Anwendungen eine höhere Stabilität auf.

Organische Carbodiimide sind bekannt und finden beispielsweise Verwendung als Stabilisator gegen den hydrolytischen Abbau von Estergruppen enthaltenden Verbindungen, beispielsweise von Polyadditions- und Polykondensationsprodukten, wie Polyurethanen. Carbodiimide können nach allgemein bekannten Verfahren hergestellt werden, beispielsweise durch Einwirkung von Katalysatoren auf Mono- oder Polyisocyanate unter Kohlendioxidabspaltung. Als Katalysatoren geeignet sind z.B. heterocyclische, Phosphor enthaltende Verbindungen, wie Phospholine, Phospholene und Phospholidine sowie deren Oxide und Sulfide und/oder Metallcarbonyle.

Derartige Carbodiimide, ihre Herstellung und Verwendung als Stabilisatoren gegen die hydrolytische Spaltung von Kunststoffen auf Polyesterbasis werden z.B. beschrieben US-A 5597942, US-A 5733959 und US-A 5 210 170.

In der DE 10 2004 041 605 A1 wird ein strukturell besonders definiertes Carbodiimid sowie Verfahren zu dessen Herstellung beschrieben. In den konkreten Beispielen erfolgt die Herstellung lösemittelfrei, d.h. in Substanz. Die Carbodiimidherstellung erfolgt dabei in Gegenwart von Katalysatoren (insbesondere in Gegenwart von Methyl-2,5-dihydrophospholen-1-oxiden und/oder 1-Methyl-2,3-dihydrophospholen-1-oxiden). Die Katalysatoren können anschließend aus dem Polycarbodiimid durch Destillation entfernt werden. Die erhaltenen (noch Isocyanatgruppen enthaltenden) Polycarbodiimide werden im Anschluss mit weiteren Acrylaten umgesetzt (Endfunktionalisierung).

Die US-A 6 498 225 betrifft Blockcopolymere, welche unter anderem einen Carbodiimidbaustein umfassen. Die Herstellung der Carbodiimide erfolgt in Gegenwart von basischen Katalysatoren bei erhöhter Temperatur unter Abspaltung von Kohlendioxid In den Beispielen wird die Herstellung der Carbodiimide in einem Lösemittel durchgeführt (Xylol).

EP 0 965 582 A offenbart spezifische Carbodiimide auf Basis von 1,3-Bis-(1-methyl-1-isocyanato-ethyl)-benzol, enthaltend 12 bis 40 Gew.-% Ethylenoxideinheiten. Die Herstellung der Carbodiimide erfolgt auf herkömmliche Weise, wie bereits oben beschrieben. Die Umsetzung kann dabei in Abwesenheit oder aber in Gegenwart von organischen Lösemitteln durchgeführt werden.

EP 0 940 389 A beschreibt strukturell spezifische Carbodiimide, welche neben der Carbodiimidfunktion Urethangruppen und/oder Harnstoffgruppen enthalten, wobei die Carbodiimidstrukturen an nichtaromatische Kohlenwasserstoffatome gebunden sind. Ferner wird ein Verfahren zur Herstellung dieser Carbodiimide sowie Mischungen, welche diese Carbodiimide enthalten, beschrieben. Die Herstellung der Carbodiimide erfolgt dabei in Abwesenheit oder in Gegenwart von organischen Lösemitteln. In den Beispielen erfolgt die Synthese frei von Lösemittel, d.h. in Substanz.

EP 1 125 956 A betrifft strukturell spezifische Carbodiimide, welche des Weiteren Harnstoffgruppen und/oder Sulfonsäuregruppen und/oder Sulfonatgruppen enthalten. Die Herstellung dieser Carbodiimide erfolgt durch Umsetzung von 1,3-Bis-(1-methyl-1-isocyanatoethyl)-benzol mit mindestens einer Aminosulfonsäure und/oder mindestens einem Aminosulfonat in Gegenwart herkömmlicher Katalysatoren, wobei die Umsetzung vorzugsweise in Lösemitteln durchgeführt wird. Nach Erreichen des gewünschten Gehalts an NCO-Gruppen wird die Polycarbodiimidbildung unterbrochen und die Katalysatoren werden abdestilliert oder deaktiviert. In den Beispielen wird ein Polycarbodiimid als Edukt eingesetzt, welches nach US-A 5 597 942 erhalten wurde; eine detailierte Darstellung der Carbodiimidherstellung erfolgt in den Beispielen nicht.

EP 0 792 897 A offenbart spezifische aromatische Polycarbodiimide und deren Herstellung auf herkömmliche Weise mittels Phosphor-haltiger Katalysatoren. Die Herstellung erfolgt in einem Lösemittel. Es kann am Anfang, in der Mitte oder am Ende der Reaktion zur Herstellung des Carbodiimids ein Isocyanat zum Kappen des Endes des Carbodiimids hinzugegeben werden. Am Ende der Reaktion wird die Reaktionsmischung in ein Lösemittel gegeben, in welches das Carbodiimid unlöslich ist, so dass es sich abscheidet und von dem Monomer und dem Katalysator abgetrennt werden kann. In den Beispielen erfolgt die Carbodiimidherstellung in Tetrahydrofuran (THF).

US 5,750,636 beschreibt die Herstellung von Polycarbodiimiden in einem Lösemittel mit herkömmlichen Katalysatoren. Das Lösemittel ist chloriert.

EP 0 686 626 A betrifft spezielle hydrophile Carbodiimide. Hinsichtlich der Synthese der Carbodiimide wird auf herkömmliche Verfahren verwiesen. In den Beispielen erfolgt die Carbodiimidbildung in Substanz.

In EP 0 628 541 A werden strukturell-spezielle Carbodiimide bzw. Oligocarbodiimide mit endständigen Isocyanat-, Harnstoff- und/oder Urethangruppen beschrieben. Bei der Herstellung der Carbodiimide kann in Gegenwart oder Abwesenheit von Lösemitteln gearbeitet werden. Endständige Isocyanate können nach der Herstellung noch blockiert werden. In den Beispielen wird bei der Carbodiimidbildung lösemittelfrei gearbeitet.

Aus dem Stand der Technik sind somit Verfahren zur Herstellung von Carbodiimiden bekannt, welche entweder in Substanz oder aber in Gegenwart von Lösemitteln stattfinden.

Die durch diese Verfahren der Kondensation in Lösung oder Substanz erhältlichen Carbodiimide weisen den Nachteil von relativ geringen Molekulargewichten auf.

Ferner weisen die aus dem Stand der Technik erhältlichen Carbodiimide eine zu hohe Polydispersität auf.

Der vorliegenden Erfindung liegt damit die Aufgabe zugrunde, ein Verfahren zur Herstellung von Carbodiimiden bereit zu stellen, mit welchem Carbodiimide erhalten werden, welche vorzugsweise höhere Molekulargewichte als die mit den herkömmlichen Verfahren erhältlichen Carbodiimide aufweisen.

Der vorliegenden Erfindung liegt ferner die Aufgabe zugrunde, ein Verfahren zur Herstellung von Carbodiimiden bereit zu stellen, mit welchem Carbodiimide erhalten werden, welche vorzugsweise geringere Polydispersitäten als die mit den herkömmlichen Verfahren erhältlichen Carbodiimide aufweisen.

Der vorliegenden Erfindung liegt ferner die Aufgabe zugrunde, ein Verfahren zur Herstellung von Carbodiimiden bereit zu stellen, mit welchem Carbodiimide erhalten werden, welche vorzugsweise höhere Molekulargewichte als die mit den herkömmlichen Verfahren erhältlichen Carbodiimide aufweisen, und mit welchem Carbodiimide erhalten werden, welche vorzugsweise geringe Polydispersitäten als die mit den herkömmlichen Verfahren erhältlichen Carbodiimide aufweisen.

Ferner liegt der vorliegenden Erfindung die Aufgabe zugrunde, ein Verfahren zur Herstellung von Carbodiimiden bereitzustellen, mit welchem es vorzugsweise möglich ist, geringe Restgehalte an Isocyanaten in den Carbodiimiden zu erreichen. Insbesondere soll der Restgehalt an Isocyanat in dem Carbodiimid weniger als 1,5 Gew.-%, bezogen auf das Carbodiimid, betragen.

Schließlich soll das erfindungsgemäße Verfahren eine mit den herkömmlichen Verfahren vergleichbare Gesamtreaktionszeit bei einem vergleichbaren Umsatz von ungefähr 95 % aufweisen.

Gelöst wird diese Aufgabe durch das erfindungsgemäße Verfahren zur Herstellung von Verbindungen, welche mindestens eine Carbodiimidgruppe umfassen, durch mindestens eine zweistufige Umsetzung von mindestens einer Isocyanat-haltigen Ausgangsverbindung in der Gegenwart eines Katalysators, dadurch gekennzeichnet, dass diese
(1) zunächst einer ersten Polymerisation in Substanz unterworfen, wobei ein erstes Polymerisationsprodukt erhalten wird; und
(2) in Verfahrensschritt (2) das aus Verfahrensschritt (1) stammende erste Polymerisationsprodukt einer zweiten Polymerisation in Lösung bei Temperaturen von 100°C bis 200°C unterworfen wird, wobei die erste Polymerisation in Verfahrensschritt (1) bis zu einem Isocyanatumsatz von 40 % bis 60 % durchgeführt wird.

In der zweiten Verfahrensstufe wird vorzugsweise kein weiterer Katalysator zugegeben.

In der zweiten Verfahrensstufe erfolgt die Lösemittelzugabe vorzugsweise ohne ein vorheriges Abkühlen.

Erfindungsgemäß wurde herausgefunden, dass ein Kombinationsverfahren zur Herstellung von Carbodiimiden, welches sowohl eine Polymerisation in Substanz (Verfahrensschritt (1)) als auch eine Polymerisation in Lösung (Verfahrensschritt (2)) umfasst, die vorstehend definierten Aufgaben löst. Insbesondere weisen die durch das erfindungsgemäße Verfahren erhältlichen Carbodiimide - im Vergleich zu den Carbodiimiden, welche entweder nur mit einem Verfahren in Substanz oder aber nur mit einem Verfahren in Lösung erhalten werden - höhere Molekularmassen und eine geringere Polydispersität auf.

Darüber hinaus können mit dem erfindungsgemäßen Verfahren Carbodiimide erhalten werden, welche einen Restgehalt an Isocyanat von vorzugsweise weniger als 2,00 Gew.-%, weiter bevorzugt weniger als 1,5 Gew.-%, insbesondere bevorzugt weniger als 1,00 Gew.-%, speziell weniger als 0,75 Gew.-%, aufweisen.

Die für das erfindungsgemäße Verfahren anzusetzende Gesamtreaktionsdauer liegt in dem Bereich der herkömmlichen Verfahren, welche aus dem Stand der Technik bekannt sind.

Im Rahmen der vorliegenden Erfindung wird unter einem herkömmlichen Verfahren zur Herstellung von Carbodiimid ein Verfahren verstanden, bei welchem eine Isocyanat-haltige Verbindung in Gegenwart eines Katalysators und unter Abspaltung von Kohlendioxid zu einem Carbodiimid umgesetzt wird und das Verfahren ausschließlich entweder in Substanz oder aber ausschließlich in Lösung durchgeführt wird.

Im Folgenden werden besondere Ausgestaltungen des erfindungsgemäßen Verfahrens beschrieben, ohne dass die vorliegende Erfindung hierauf beschränkt ist:
Die Zugabe des Lösemittels zur Durchführung der Polymerisation in Lösung (d.h. Verfahrensschritt (2)) erfolgt nach einem Isocyanatumsatz von 40 bis 60 %, vorzugsweise 45 bis 55 %, insbesondere bei ungefähr 50 %. Das erfindungsgemäße Verfahren wird im zweiten Verfahrensschritt bis zu einem NCO-Gehalt der Verbindungen, die mindestens eine Carbodiimdgruppe umfassen, von vorzugsweise höchstens 2,00 Gew.-%, bevorzugt höchstens 1,5 Gew.-%, besonders bevorzugt höchstens 1,00 Gew.-%, speziell höchstens 0,75 Gew.-%, durchgeführt.

Im Rahmen der vorliegenden Erfindung kann als Isocyanat-haltige Verbindung jede beliebige Verbindung verwendet werden, welche mindestens eine Isocyanatgruppe umfasst. Insbesondere wird die Isocyanat-haltige Verbindung ausgewählt aus der Gruppe, bestehend aus Cyclohexylisocyanat, Isophorondiisocyanat (IPDI), Hexamethylendiisocyanat (HDI), 2-Methylpentandiisocyanat (MPDI), Methylenbis-(2,6-diisopropylphenylisocyanat) (MDIPI); 2,2,4-Trimethylhexamethylendiisocyanat/2,4,4-Trimethyl-hexamethylendiisocyanat (TMDI), Norbornandiisocyanat (NBDI), Methylendiphenyldiisocyanat (MDI), Diisocyanatomethylbenzen, insbesondere die 2,4- und das 2,6-Isomere und technischen Gemische beider Isomeren (TDI), Tetramethylxylylendiisocyanat (TMXDI), 1,3-Bis-(1-methyl-1-isocyanato-ethyl)-benzol, 1,3,5-Triisopropyl-2,4-diisocyanatobenzol (TRIDI) und/oder Dicyclohexylmehtyldiisocyanat (H12MDI).

Im Rahmen der vorliegenden Erfindung ist die Isocyanat-haltige Verbindung insbesondere ausgewählt aus der Gruppe, bestehend aus Isophorondiisocyanat (IPDI), Tetramethylxylylendiisocyanat (TMXDI), Dicyclohexylmehtyldiisocyanat (H12MDI) und 1,3,5-Triisopropyl-2,4-diisocyanatobenzol (TRIDI).

Im Rahmen der vorliegenden Erfindung ist die Isocyanat-haltige Verbindung noch weiter bevorzugt 1,3,5-Triisopropyl-2,4-diisocyanatobenzol (TRIDI).

Die Kondensationsreaktion der mindestens einen Isocyanat-haltigen Verbindung bedarf im Allgemeinen eines Katalysators.

In einer ersten Ausgestaltung des erfindungsgemäßen Verfahrens handelt es sich bei dem Katalysator um mindestens einen Phosphor-haltigen Katalysator. Des Weiteren kann der Katalysator ausgewählt sein aus der Gruppe, bestehend aus Phospholenen, Phospholenoxiden, Phospholidinen und Phospholinoxiden. Der für die Umsetzung der mindestens einen Isocyanat-haltigen Ausgangsverbindung verwendete Katalysator wird noch weiter bevorzugt ausgewählt aus der Gruppe, bestehend aus Methyl-2,5-dihydro-phospholen-1-oxid (CAS [930-38-1]), 1-Methyl-2,3-dihydrophospholen-1-oxid (CAS [872-45-7]) und Mischungen davon (CAS [31563-86-7)].

In einer zweiten Ausgestaltung des erfindungsgemäßen Verfahrens handelt es sich bei dem Katalysator um eine Base. Die Base ist vorzugsweise ausgewählt aus der Gruppe, bestehend aus Alkalihydroxide, beispielsweise KOH oder NaOH; Alkoholate, beispielsweise Kalium-tert.-butylat, Kaliummethylat und Natriummethylat.

Im Rahmen der vorliegenden Erfindung beträgt in der ersten Ausgestaltung der Gehalt an Katalysator vorzugsweise 0,001 bis 1,00 Gew.-%, bevorzugt 0,001 bis 0,50 Gew.-%, besonders bevorzugt 0,001 bis 0,02 Gew.-%, jeweils bezogen auf die mindestens eine Isocyanat-haltige Verbindung.

Im Rahmen der vorliegenden Erfindung beträgt in der zweiten Ausgestaltung der Gehalt an Katalysator vorzugsweise 0,01 bis 2,00 Gew.-%, bevorzugt 0,05 bis 1,00 Gew.-%, besonders bevorzugt 0,1 bis 0,50 Gew.-%, jeweils bezogen auf die mindestens eine Isocyanat-haltige Verbindung.

Im Verfahrensschritt (2) des erfindungsgemäßen Verfahrens, d.h. während der Polymerisation in Lösung, wird ein Lösemittel verwendet, welches vorzugsweise ausgewählt wird aus der Gruppe, bestehend aus Benzol; Alkylbenzol, insbesondere und vorzugsweise Toluol, o-Xylol, m-Xylol, p-Xylol, Diisoproypbenzol und/oder Triisopropylbenzol; Aceton; Isobutylmethylketon; Tetrahydrofuran; Hexan; Benzin; Dioxan; N-Methylpyrrolidon; Dimethylformamid und/oder Dimethylacetamid.

In dem Verfahrensschritt (2) werden im Allgemeinen, jeweils bezogen auf die in Verfahrensschritt (1) eingesetzte Monomermenge, 0,5 bis 80 Gew.-%, besonders bevorzugt 1 bis 50 Gew.-%, insbesondere 2 bis 20 Gew.-%, Lösemittel zugegeben.

Durch geeignete Wahl der Reaktionsbedingungen, z.B. der Reaktionstemperatur, der Katalysatorart und der Katalysatormenge sowie der Reaktionszeit, kann der Fachmann in der üblichen Weise den Kondensationsgrad einstellen. Der Verlauf der Reaktion kann am einfachsten durch Bestimmung des NCO-Gehaltes verfolgt werden. Auch andere Parameter, z.B. Viskositätsanstieg, Farbvertiefung oder CO₂-Entwicklung, kann man für die Verfolgung des Ablaufs und die Steuerung der Reaktion heranziehen.

Hinsichtlich der Temperaturen, bei welcher die Carbodiimidbildung durchgeführt wird, ist es bevorzugt, dass der Verfahrensschritt (1) bei einer Temperatur zwischen 50 bis 220 °C, weiter bevorzugt zwischen 100 und 200 °C, besonders bevorzugt zwischen 140 und 190 °C durchgeführt wird.

Die entsprechende Temperatur, bei welcher die Carbodiimidbildung durchgeführt wird, kann dabei mittels einer Temperaturrampe erreicht werden. Unter dem Begriff einer Temperaturrampe wird im Rahmen der vorliegenden Erfindung verstanden, dass die Temperatur, bei welcher die Carbodiimidbildung erfolgt, nicht sofort, sondern durch langsames Erwärmen erreicht wird.

Die Temperaturrampe kann beispielsweise 1 bis 10 °C/30 Minuten betragen.

Der Verfahrensschritt (2) wird bei einer Temperatur zwischen 100 und 200 °C, besonders bevorzugt zwischen 140 und 190 °C durchgeführt.

In einer weiteren Ausgestaltung der vorliegenden Erfindung wird die Umsetzung der mindestens einen Isocyanat-haltigen Verbindung in Gegenwart einer Schutzgasatmosphäre durchgeführt. Dabei kann entweder nur der erste Verfahrensschritt der Umsetzung in Substanz oder der zweite Verfahrensschritt der Umsetzung in Lösung in Gegenwart einer Schutzgasatmosphäre durchgeführt werden.

In einer bevorzugten Ausführungsform werden allerdings beide Verfahrensschritte in Gegenwart eines Schutzgases durchgeführt. Als Schutzgas wird vorzugsweise Argon, Stickstoff oder eine beliebige Mischung dieser Gase verwendet.

Wenn die Reaktionsmischung den gewünschten Gehalt an NCO-Gruppen besitzt, wird die Polycarbodiimidbildung üblicherweise beendet. Hierzu können die Katalysatoren im Fall der Verwendung von Phosphor-haltigen Katalysatoren unter vermindertem Druck abdestilliert oder durch Zusatz eines Desaktivators, wie z.B. Phosphortrichlorid, desaktiviert werden. Bevorzugt werden die Katalysatoren abdestilliert.

In einer weiteren Ausgestaltung der vorliegenden Erfindung umfasst das erfindungsgemäße Verfahren demnach nach dem Verfahrensschritt (2) einen Verfahrensschritt (3), in welchem
(3.1) mindestens ein Katalysator destillativ aus der Carbodiimidverbindung entfernt wird und/oder
(3.2) mindestens ein Katalysator durch Zusatz eines Desaktivators, wie z.B. Phosphortrichlorid, desaktiviert wird.

Gegebenenfalls wird die Reaktionsmischung auch folgender Aufarbeitung (Verfahren 3.3) unterworfen, wobei eine Abtrennung bzw. Desaktivierung des Katalysators gemäß den vorstehenden Verfahrensschritten (3.1) und/oder (3.2) vor der Aufarbeitung erfolgen kann.

Zu diesem Zweck kann zu der aus dem Verfahrensschritt (2) erhältlichen Reaktionsmischung in Abhängigkeit der Menge an Lösemittel, welches bereits im Verfahrensschritt (2) zugegeben wurde, weiteres Lösemittel zugegeben werden. Alternativ kann man, wenn ausreichend Lösemittel bereits in Verfahrensschritt (2) zugegeben wurde, auf die Zugabe von weiterem Lösemittel auch verzichten.

Wenn bei der Aufarbeitung ein Lösemittel nach dem Verfahrensschritt (2) zugegeben wird, so kann es sich um das gleiche Lösemittel handeln, welches auch bei der Lösemittelpolymerisation in Verfahrensschritt (2) verwendet wird und bereits zuvor beschrieben wurde. Insoweit wird hierauf Bezug genommen.

Im Anschluss wird das Carbodiimid vorzugsweise durch Zugabe eines polaren Lösemittels, wie Aceton, Ethylacetat, Ethanol oder Methanol ausgefällt.

Diese Aufarbeitung (Variante 3.3) ist insbesondere bevorzugt.

Eine weitere Aufarbeitung besteht darin, dass aus dem Verfahrensschritt (2) stammende Polymerisationsprodukt direkt einer Entfernung des Lösemittels zu unterwerfen und den erhaltenen Rückstand als Endprodukt zu konfektionieren.

Bevorzugt sind erfindungsgemäß Carbodiimide, die einen Gehalt an Katalysatoren für die Carbodiimidbildung von kleiner 25 ppm im Endprodukt, d.h. nach der Aufarbeitung, aufweisen.

Das Molekulargewicht von den erfindungsgemäß erhaltenen Carbodiimiden ist höher als das Molekulargewicht von entsprechenden Carbodiimiden, welche durch reine Substanzpolymerisation oder reine Polymerisation in Lösung erhalten werden, was durch die nachstehend beschriebenen Beispiele verdeutlicht wird.

Die physikalischen, mechanischen und rheologischen Eigenschaften von Carbodiimiden werden durch die Polymolekularität (das Verhältnis von Gewichtsmittel zu Zahlenmittel) bestimmt. Dieses Verhältnis wird auch als Polydispersität D bezeichnet und ist ein Maß für die Breite einer Molmassenverteilung. Je größer die Polydispersität ist, desto breiter ist auch die Molmassenverteilung. Die Bestimmung der Molekulargewichte erfolgte vorzugsweise durch Gelpermeationschromatographie in Tetrahydrofuran bei 40 °C gegen Polystyrol als Standard. Für die Messungen wurden beispielsweise drei hintereinander geschaltete Säulen (bestehend aus einem mit Divinylbenzol vernetztem Polystyrol) der Firma PSS Polymer Standards Service GmbH mit einer Partikelgröße von 5 µm und Porengrößen zwischen 100 und 500 Å bei einer Flussrate von 1 mL/ min verwendet.

Die nach dem erfindungsgemäßen Verfahren hergestellten Carbodiimide zeichnen sich durch eine Polydispersität von weniger als 2,5, bevorzugt weniger als 2,25, besonders bevorzugt weniger als 2,00, speziell zwischen 1,6 und 1,8 aus.

Die nach dem erfindungsgemäßen Verfahrne hergestellten Carbodiimide weisen ein Molekulargewicht M_{w} von 20000 bis 40000 g/mol, bevorzugt 25000 bis 35000 g/mol, besonders bevorzugt 26000 bis 34000 g/mol, auf. Die nach dem erfindungsgemäßen Verfahren erhaltenen Carbodiimide können ferner auch endfunktionalisiert werden. Entsprechende Endfunktionalisierungen sind in der DE 10 2004 041 605 A1 beschrieben, dessen diesbezügliche Offenbarung durch Bezugnahme in die vorliegende Erfindung durch Bezugnahme eingeschlossen wird.

Nach beendeter Carbodiimidisierung können in den nach dem erfindungsgemäßen Verfahren hergestellten Carbodiimide die freien endständigen Isocyanatgruppen des Carbodiimids und/oder der oligomeren Polycarbodiimide mit C-H- oder N-H-reaktiven Wasserstoffverbindungen somit blockiert oder mit aliphatischen, cycloaliphatischen und/oder araliphatischen Aminen, derartigen Alkoholen und/oder Alkoxypolyoxyalkylenalkoholen vollständig oder teilweise abgesättigt werden. Nach einer vorteilhaften Ausführungsform werden zur vollständigen Absättigung der Isocyanatgruppen die aliphatischen, cycloaliphatischen oder araliphatischen Amine, Alkohole und/oder Alkoxypolyoxyalkylenalkohole vorzugsweise in einem geringen Überschuss von -OH-, -NH- und/oder -NH₂-Gruppen zu NCO-Gruppen der (Poly)carbodiimide enthaltenden Reaktionsmischung hinzugefügt, dort ausreagieren gelassen und danach gegebenenfalls die nicht umgesetzte Menge, bevorzugt unter vermindertem Druck abdestilliert.

Nach einer anderen, vorzugsweise angewandten Verfahrensvariante können die erfindungsgemäßen (Poly)carbodiimide mit teilweise oder vollständig abgesättigten Isocyanatgruppen in der Weise hergestellt werden, dass man zunächst bis zu 50 Gew.-%, vorzugsweise bis zu 23 Gew.-% der Isocyanatgruppen mit mindestens einem aliphatischen, cycloaliphatischen oder araliphatischen Amin, Alkohol und/oder Alkoxypolyoxyalkylenalkohol umsetzt und danach die freien Isocyanatgruppen in Gegenwart von Katalysatoren unter Kohlendioxidabspaltung ganz oder teilweise zu Carbodiimiden und/oder oligomeren Polycarbodiimiden kondensiert.

Die nach dem erfindungsgemäßen Verfahren hergestellten Monocarbodiimide und/oder oligomeren Polycarbodiimide eignen sich hervorragend als Akzeptor für Carboxylverbindungen und finden daher vorzugsweise Verwendung als Stabilisatoren gegen den hydrolytischen Abbau von Estergruppen enthaltenden Verbindungen, beispielsweise Estergruppen enthaltende Polymere, z.B. Polykondensationsprodukte wie beispielsweise thermoplastische Polyester wie Polyethylen- und -butylenterephthalat, Polyetherester, Polyamide, Polyesteramide, Polycaprolactone sowie ungesättigte Polyesterharze und Polyesterester wie z.B. Blockcopolymere aus Polyethylen- oder Butylenterephthalat und Polycaprolacton und Polyadditionsprodukte, z.B. Polyurethane, Polyharnstoffe und Polyurethan-Polyharnstoff-Elastomere, die Estergruppen enthalten. Diese Estergruppen enthaltenden Verbindungen sind allgemein bekannt. Ihre Ausgangssubstanzen, Herstellverfahren, Strukturen und Eigenschaften sind in der Standardliteratur vielfältig beschrieben. Aufgrund der guten Löslichkeit in den Aufbaukomponenten zur Herstellung von Polyurethanen und der guten Verträglichkeit mit den gebildeten Polyurethanen eignen sich die erfindungsgemäßen (Poly)carbodiimide insbesondere als Stabilisatoren gegen den hydrolytischen Abbau von Polyurethanen, vorzugsweise kompakten oder zelligen Polyurethan-Elastomeren und insbesondere thermoplastischen Polyurethanen sowie zelligen oder kompakten Elastomeren.

Insbesondere werden die nach dem erfindungsgemäßen hergestellten Carbodiimide zur Herstellung von Kunststofffolien, insbesondere PET (Polyethylenterephalat)-Folien, TPU (Thermoplastische Polyurethan)-Folien und PLA(Polymilchsäure)-Folien verwendet.

Die vorliegende Erfindung wird anhand der nachfolgenden Beispiele näher erläutert, ohne dabei limitierend zu sein.

### Ausführungsbeispiele:

Es wurden insgesamt vier Verfahren zur Herstellung von Carbodiimiden durchgeführt:

### Versuch a): Substanzpolymerisation

In einem ausgeheizten und mit Stickstoff befüllten 500 mL Planschliffkolben werden unter Stickstoff 306 g Triisopropylbenzyldiisocyanat vorgelegt und auf 140 °C erwärmt. Nach Zugabe von 19 mg 1-Methyl-phospholenoxid wird das Reaktionsgemisch innerhalb von 5 Stunden auf 180 °C erhitzt. Bei dieser Temperatur wird 43 Stunden reagieren gelassen und ein NCO-Gehalt von 2,1 % (entspricht 93 % Umsatz) erreicht.

### Versuch b): Lösungspolymerisation

In einem ausgeheizten und mit Stickstoff befüllten 500 mL Planschliffkolben werden unter Stickstoff 420 g einer 50 %-igen Lösung Triisopropylbenzoldiisocyanat in Diisopropylbenzol vorgelegt und auf 140 °C erwärmt. Nach Zugabe von 0,005 % MPO (12 mg) bezogen auf das Isocyanat wird das Reaktionsgemisch innerhalb von 5 Stunden auf 180 °C erhitzt. Bei dieser Temperatur wird 43 Stunden reagieren gelassen und ein NCO-Gehalt von 3,4 % (entspricht 77 % Umsatz) erreicht.

### Versuch c): Kombinationsverfahren

In einem ausgeheizten und mit Stickstoff befüllten 500 mL Planschliffkolben werden unter Stickstoff 420 g Triisopropylbenzyldiisocyanat vorgelegt und auf 140 °C erwärmt. Nach Zugabe von 24 mg 1-Methyl-phospholenoxid wird das Reaktionsgemisch innerhalb von 5 Stunden auf 180 °C erhitzt. Nach 3 Stunden bei dieser Temperatur werden 48 g Diisopropylbenzol zugegeben. Im Anschluss wird weitere 40 Stunden bei 180 °C reagieren gelassen und ein NCO-Gehalt von 1,1 % (entspricht 96 % Umsatz) erreicht.

### Auswertung:

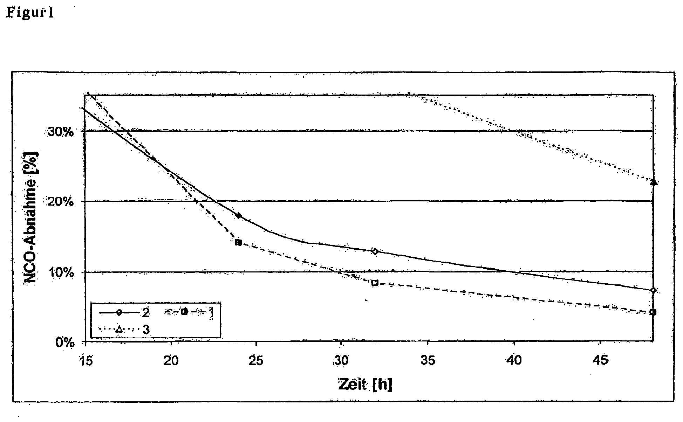

Da der Isocyanatgehalt pro Gramm Substanz ermittelt wird, berücksichtigen zur besseren Vergleichbarkeit die in der Figur 1 dargestellten Isocyanatgehälter bereits die unterschiedlichen Lösungsmittelmengen.

Die Abnahme im NCO-Gehalt zeigt, dass die Polymerisation in einer 50 %-igen Lösung die langsamste Reaktion ist (Vergleich: Lösungspolymerisation). Nach 48 Stunden sind noch knapp 23 % der Startmenge NCO vorhanden.

In den beiden anderen Fällen handelt es sich für die ersten 8 Stunden um eine Polymerisation in Substanz. Die stärkere Abnahme der Reaktionsgeschwindigkeit wird nach ca. 15 Stunden deutlich; vgl. Figur 1. So sind nach 48 Stunden Reaktionszeit in Substanz noch 7,2 % der Ausgangskonzentration Isocyanat vorhanden, während im Kombinationsverfahren nur noch 4,1 % vorliegen.

Der NCO-Verlauf über eine Reaktionszeit von 15 bis 48 Stunden ist in Figur 1 dargestellt. Dabei zeigt die Kurve für (1) die Abnahme des NCO-Gehaltes für das erfindungsgemäße Kombi-Verfahren (Quadrat)). Zum Vergleich dienen (2) die Umsetzung in Substanz (Raute) und (3) die Umsetzung in Lösung (Dreieck).

Hierdurch wird deutlich, dass das erfindungsgemäße Verfahren schneller verläuft als die herkömmlichen Verfahren der Substanz- bzw. reinen Lösemittelpolymerisation.

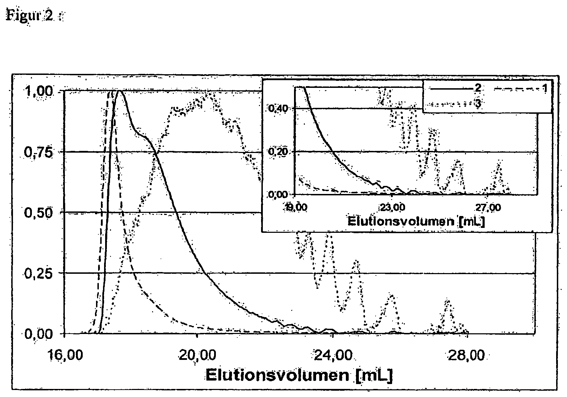

In Figur 2 sind die Elutionsdiagramme (in THF gegen Polystyrol als Standard gemessen) der Verfahrensvarianten der Carbodiimidisierungen in Substanz (Vergleich, durchgezogene Linie (2)), in Lösung (Vergleich, gepunktete Linie (3)) und im erfindungsgemäßen Kombinationsverfahren (gestrichelte Linie, (1)) abgebildet.

Das Lösungsmittelsignal wurde abgeschnitten.

Ein Vergleich der Kurven zeigt, dass im erfindungsgemäßen Kombinationsverfahren das Maximum bei einem Elutionsvolumen von ca. 17,4 mL liegt, wohingegen im Substanzverfahren zwei Spitzen bei höherem Elutionsvolumen (17,8 mL und 18,6 mL) zu verzeichnen sind. Diese Beobachtung spiegelt sich auch in den erreichten gewichtsmittleren Molmassen wieder. So beträgt die gewichtsmittlere Molmasse in der Substanzpolymerisation 13800 g/mol während in Lösung nur eine gewichtsmittlere Molmasse von 4600 g/ mol erreicht wird. Das erfindungsgemäße Kombinationsverfahren liefert nach der gleichen Zeit ein Gewichtsmittel von 28900 g/mol und ist damit etwa doppelt so groß wie das der Substanzpolymerisation. Tabelle 2 können sowohl die gewichtsmittleren, die zahlenmittleren Molmassen sowie die Polydispersität für die drei Verfahrensvarianten entnommen werden. Die Chromatogramme zeigen bereits, dass die Molmassenverteilung nach dem Kombinationsverfahren am geringsten ist. Dieser optische Eindruck wird durch die Werte der Polydispersitäten, wie sie in Tabelle 2 aufgezeigt sind, bestätigt.

**Tabelle 1: NCO-Abnahme/ freies Isocyanat im Verlauf der Reaktion bei den drei Verfahrensvarianten. Zum besseren Vergleich wurde der Lösungsmittelanteil nicht berücksichtigt.**

| Zeit [h] | Substanz (Vergleich) | Lösung (Vergleich) | Kombination (erfindungsgemäß) |
|---|---|---|---|
| 0 | 100,00% | 100,00% | 100,00% |
| 2 | 86,70% | 92,11% | 92,18% |
| 4 | 68,78% | 84,69% | 81,29% |
| 6 | 59,52% | 79,73% | 63,27% |
| 8 | 46,53% | 71,29% | 52,38% |
| 24 | 17,93% | 44,76% | 14,02% |
| 32 | 12,82% | 37,01% | 8,23% |
| 48 | 7,21% | 22,79% | 4,12% |

**Tabelle 2: GPC-Daten der drei Verfahrensvarianten nach Messungen in THF gegen Polystyrol als Standard.**

| **Verfahren** | **Mn [g/mol]** | **Mw [g/mol]** | **D** |
|---|---|---|---|
| Substanz (Vergleich) | 4900 | 13800 | 2,78 |
| Lösungsmittel (Vergleich) | 1500 | 4600 | 2,99 |
| Kombination (erfindungsgemäß) | 13300 | 28900 | 2,18 |

### Versuch d): Kombinationsverfahren (erfindungsgemäß)

In einem ausgeheizten und mit Stickstoff befüllten 500 mL Planschliffkolben werden unter Stickstoff 420 g Triisopropylbenzyldiisocyanat vorgelegt und auf 140 °C erwärmt. Nach Zugabe von 26mg 1-Methyl-phospholenoxid wird das Reaktionsgemisch innerhalb von 5 Stunden auf 180 °C erhitzt. Nach 1,5 Stunden bei dieser Temperatur werden 45 g Diisopropylbenzol zugegeben. Im Anschluss wird weitere 40 Stunden bei 180 °C reagieren gelassen und ein NCO-Gehalt von 0,8 % (entspricht 97 % Umsatz) erreicht.

In Versuch d) liegt der NCO-Gehalt vor der Zugabe des Lösungsmittels bei 15,4 % und der bis dahin aufgebaute NCN-Gehalt bei 8,75 %.

Nach den 48 Stunden Reaktionszeit beträgt der NCO-Gehalt noch 0,8 %, wobei der NCN-Gehalt bis auf 12,9 % (Lösungsmittel ist in der Probe noch enthalten) angewachsen ist.

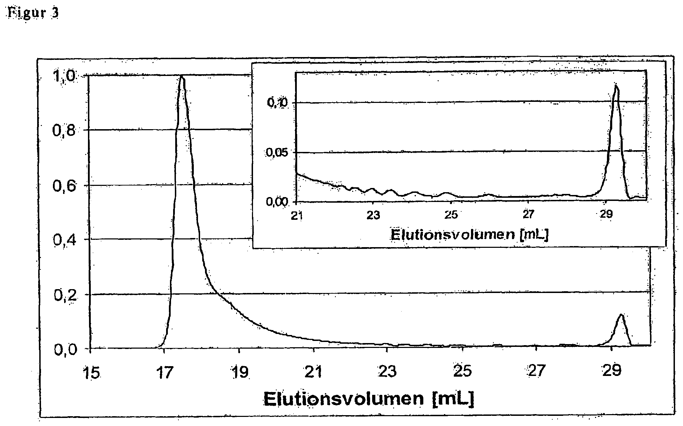

Figur 3 zeigt das Chromatogramm der Probe nach 48 Stunden Reaktionszeit. Das Signal bei ca. 29 mL stammt vom Lösungsmittel. Die zahlen- und gewichtsmittleren Molekulargewichte mit und ohne Lösungsmittelsignal sowie die daraus resultierenden Polydispersitäten können Tabelle 3 entnommen werden.

**Tabelle 3: Ergebnisse der GPC in THF und gegen Polystyrol als Standard von Versuch d) nach einer Reaktionszeit von 48 Stunden.**

| Auswertung: | Mn **[g/mol]** | Mw **[g/mol]** | D |
|---|---|---|---|
| mit Lösungsmittel | 3300 | 29600 | 9,08 |
| ohne Lösungsmittel | 13500 | 30900 | 2,29 |

### Aufarbeitung 1:

Durch vierstündige Destillation bei 180 °C im Vakuum konnte ein Großteil des Lösungsmittels entfernt werden.

Nach der Destillation beträgt der NCO-Gehalt noch 0,6 %, wobei der NCN-Gehalt auf 13,6 % (Rest-Lösungsmittel ist in der Probe noch enthalten) angewachsen ist.

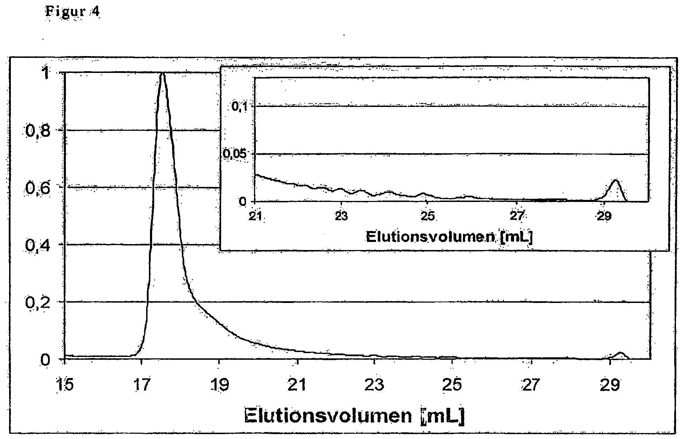

Figur 4 zeigt das Gelpermeationschromatogramm der Probe nach 52 Stunden Reaktionszeit(48 Stunden Reaktionszeit und vier Stunden destillative Entfernung des Lösungsmittels). Das Signal bei ca. 29 mL stammt vom Lösungsmittel und ist im Vergleich zur Probe vor der Destillation deutlich gesunken. Die zahlen- und gewichtsmittleren Molekulargewichte mit und ohne Lösungsmittelsignal sowie die daraus resultierenden Polydispersitäten können Tabelle 4 entnommen werden.

**Tabelle 4: Ergebnisse der GPC in THF und gegen Polystyrol als Standard von Versuch d) nach einer Reaktionszeit von 48 Stunden und vier Stunden destillativer Entfernung des Lösungsmittels.**

| Auswertung: | Mn **[g/mol]** | Mw **[g/mol]** | D |
|---|---|---|---|
| mit Lösungsmittel | 8500 | 31800 | 3,76 |
| ohne Lösungsmittel | 15100 | 32100 | 2,13 |

### Aufarbeitung 2 - Fällung 1

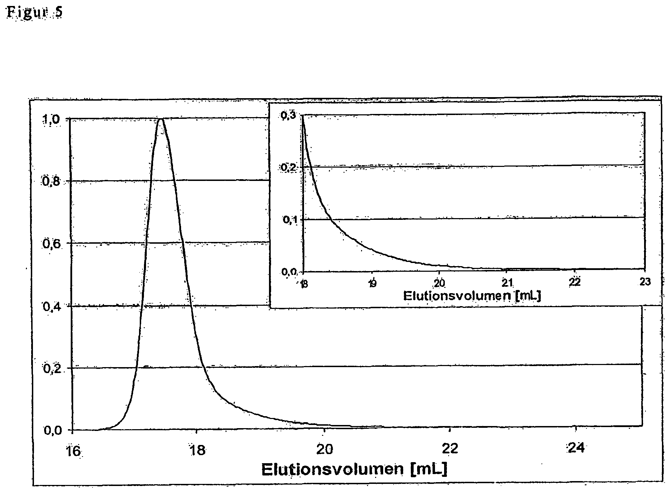

Das erhaltene Produkt nach 48 Stunden Reaktionszeit wird als 54 %-ige toluolische Lösung bei 85 °C hergestellt. Diese Lösung wird langsam in die 1,4-fache Menge Aceton gegeben und das ausgefallene Produkt filtriert, gewaschen und getrocknet. Der Verlauf des Elutionsvolumens ist in Figur 5 dargestellt.

**Tabelle 5: Ergebnisse der GPC in THF und gegen Polystyrol als Standard von Versuch d) nach einer Reaktionszeit von 48 Stunden nach der Fällung aus Aceton.**

| | Mn **[g/mol]** | Mw **[g/mol]** | D |
|---|---|---|---|
| Versuch d Fällung 1 | 19370 | 34170 | 1,76 |

## Patentansprüche

1. Verfahren zur Herstellung von Verbindungen, welche mindestens eine Carbodiimidgruppe umfassen, durch mindestens eine zweistufige Umsetzung von mindestens einer Isocyanat-haltigen Ausgangsverbindung in der Gegenwart eines Katalysators, **dadurch gekennzeichnet, dass** die
(1) zunächst einer ersten Polymerisation in Substanz unterworfen werden, wobei ein erstes Polymerisationsprodukt erhalten wird; und
(2) das aus Verfahrensschritt (1) stammende erste Polymerisationsprodukt einer zweiten Polymerisation in Lösung bei Temperaturen von 100 bis 200°C unterworfen wird,
wobei die erste Polymerisation in Verfahrensschritt (1) bis zu einem Isocyanatumsatz von 40 bis 60 % durchgeführt wird.

2. Verfahren nach Anspruch 1, **dadurch gekennzeichnet, dass** die Isocyanat-haltige Verbindung ausgewählt ist aus der Gruppe, bestehend aus Cyclohexylisocyanat, Isophorondiisocyanat (IPDI), Hexamethylen-diisocyanat (HDI), 2-Methylpentandiisocyanat (MPDI), Methylenbis-(2,6-diisopropylphenylisocyanat) (MDIPI); 2,2,4-Trimethylhexamethylendiisocyanat/2,4,4-Trimethyl-hexamethylendiisocyanat (TM-DI), Norbornandiisocyanat (NBDI), Methylendiphenyldiisocyanat (MDI), Diisocyanatomethylbenzen, sowie die 2,4- und das 2,6-Isomere und technischen Gemischen beider Isomeren (TDI), Tetramethylxylylendiisocyanat (TMXDI), 1,3-Bis-(1-methyl-1-isocyanatoethyl)-benzol, 1,3,5-Triisopropyl-2,4-diisocyanatobenzol (TRIDI) und/oder Dicyclohexylmethyldiisocyanat (H12MDI).

3. Verfahren nach einem der Ansprüche 1 bis 2, **dadurch gekennzeichnet, dass** der Verfahrensschritt (1) bei einer Temperatur zwischen 50 bis 220°C durchgeführt wird.

4. Verfahren nach Anspruch 3, **dadurch gekennzeichnet, dass** die gemäß Anspruch 3 definierte Temperatur mittels einer Temperaturrampe erreicht wird.

5. Verfahren nach einem der Ansprüche 1 bis 4, **dadurch gekennzeichnet, dass** das in Verfahrensschritt (2) verwendete Lösemittel ausgewählt wird aus der Gruppe, bestehend aus Benzol; Alkylbenzol, insbesondere Toluol, o-Xylol, m-Xylol, p-Xylol, Diisopropylbenzol und/oder Triisopropylbenzol; Aceton; Isobutylmethylketon; Tetrahydrofuran; Hexan; Benzin; Dioxan; N-Methylpyrrolidon; Dimethylformamid und/oder Dimethylacetamid.

## Claims

1. Process for preparing compounds which comprise at least one carbodiimide group by at least reacting at least one isocyanate-containing starting compound in two stages in the presence of a catalyst, **characterized in that** they
(1) are subjected first to a first polymerization in bulk, to give a first polymerization product; and
(2) the first polymerization product, originating from process step (1), is subjected to a second polymerization in solution at temperatures of from 100 to 200°C wherein the first polymerization in process step (1) is carried out to an isocyanate conversion of 40% to 60%.

2. Process according to Claim 1, **characterized in that** the isocyanate-containing compound is selected from the group consisting of cyclohexyl isocyanate, isophorone diisocyanate (IPDI), hexamethylene diisocyanate (HDI), 2-methylpentane diisocyanate (MPDI), methylenebis(2,6-diisopropylphenyl isocyanate) (MDIPI); 2,2,4-trimethylhexamethylene diisocyanate/2,4,4-trimethylhexamethylene diisocyanate (TMDI), norbornane diisocyanate (NBDI), methylenediphenyl diisocyanate (MDI), diisocyanatomethylbenzene, and also the 2,4- and the 2,6-isomer and technical mixtures of both isomers (TDI), tetramethylxylylene diisocyanate (TMXDI), 1,3-bis(1-methyl-1-isocyanatoethyl)benzene, 1,3,5-triisopropyl-2,4-diisocyanatobenzene (TRIDI) and/or dicyclohexylmethyl diisocyanate (H12MDI).

3. Process according to either of Claims 1 and 2, **characterized in that** process step (1) is carried out at a temperature between 50 to 220°C.

4. Process according to Claim 3, **characterized in that** the temperature defined in Claim 3 is achieved by means of a temperature ramp.

5. Process according to any of Claims 1 to 4, **characterized in that** the solvent used in process step (2) is selected from the group consisting of benzene, alkylbenzene, more particularly toluene, o-xylene, m-xylene, p-xylene, diisopropylbenzene and/or triisopropylbenzene; acetone; isobutyl methyl ketone; tetrahydrofuran; hexane; benzine; dioxane; N-methylpyrrolidone; dimethylformamide and/or dimethylacetamide.

## Revendications

1. Procédé de fabrication de composés, qui comprennent au moins un groupe carbodiimide, par au moins une réaction à deux étapes d'au moins un composé de départ contenant un groupe isocyanate en présence d'un catalyseur, **caractérisé en ce qu'**ils
(1) sont tout d'abord soumis à une première polymérisation en substance, un premier produit de polymérisation étant obtenu ; et
(2) le premier produit de polymérisation issu de l'étape de procédé (1) est soumis à une seconde polymérisation en solution à des températures de 100 à 200°C,
la première polymérisation à l'étape de procédé (1) étant réalisée jusqu'à une conversion de l'isocyanate de 40 à 60 %.

2. Procédé selon la revendication 1, **caractérisé en ce que** le composé contenant un groupe isocyanate est choisi dans le groupe constitué par l'isocyanate de cyclohexyle, le diisocyanate d'isophorone (IPDI), le diisocyanate d'hexaméthylène (HDI), le diisocyanate de 2-méthylpentane (MPDI), le bis-(2,6-diisopropylphénylisocyanate) de méthylène (MDIPI) ; le diisocyanate de 2,2,4-triméthylhexaméthylène/diisocyanate de 2,4,4-triméthylhexaméthylène (TMDI), le diisocyanate de norbornane (NBDI), le diphényldiisocyanate de méthylène (MDI), le diisocyanatométhylbenzène, ainsi que les isomères 2,4 et 2,6 et les mélanges techniques des deux isomères (TDI), le diisocyanate de tétraméthylxylylène (TMXDI), le 1,3-bis-(1-méthyl-1-isocyanato-éthyl)-benzène, le 1,3,5-triisopropyl-2,4-diisocyanatobenzène (TRIDI) et/ou le diisocyanate de dicyclohexylméthyle (H12MDI).

3. Procédé selon l'une quelconque des revendications 1 à 2, **caractérisé en ce que** l'étape de procédé (1) est réalisée à une température comprise entre 50 et 220 °C.

4. Procédé selon la revendication 3, **caractérisé en ce que** la température définie selon la revendication 3 est atteinte au moyen d'une rampe de température.

5. Procédé selon l'une quelconque des revendications 1 à 4, **caractérisé en ce que** le solvant utilisé à l'étape de procédé (2) est choisi dans le groupe constitué par le benzène ; l'alkylbenzène, notamment le toluène, l'o-xylène, le m-xylène, le p-xylène, le diisopropylbenzène et/ou le triisopropylbenzène ; l'acétone ; l'isobutylméthylcétone ; le tétrahydrofurane ; l'hexane ; l'essence ; le dioxane ; la N-méthylpyrrolidone ; le diméthylformamide et/ou le diméthylacétamide.
